# EUROPEAN PATENT APPLICATION

(11) **EP 4 467 124 A1**
(43) Date of publication of application: **27.11.2024**
(21) Application number: 22923667.4
(22) Date of filing: 30.12.2022
(51) Int. Cl.: A61K 6/80, A61K 6/891, A61K 6/20, A61K 8/64, A61Q 11/00, A61P 1/02

(54) **MINERALIZATION MATERIAL FOR TREATING TOOTH SENSITIVITY AND PROMOTING ENAMEL EPITAXIAL GROWTH AND USE THEREOF**

(30) Priority: 28.01.2022 CN 202210109256
(71) Applicant: Shaanxi Normal University, Xi'an, Shaanxi 710062 (CN)
(72) Inventor: YANG, Peng, Xi'an, Shaanxi 710062 (CN); HU, Bowen, Xi'an, Shaanxi 710062 (CN)
(74) Representative: LLR
(86) International application number: PCT/CN2022/143803
(87) International publication number: WO 2023/142882

(57) **Abstract**

The present application discloses a biomineralization material for treating dentin hypersensitivity and promoting epitaxial growth of enamel and a use thereof. The biomineralization material includes the following raw materials in parts by weight: 5-72 parts of a film-forming protein or a PEGylated film-forming protein, 4-67 parts of a water-soluble disulfide bond exchanger and 1-21 parts of a pH regulator. The present application proposes for the first time that the disulfide bond exchanger (e.g., cysteine (Cys)) without any toxicity may react with the film-forming protein or PEGylated film-forming protein. When a protein nano-coating with a mineralization regulating function is formed, no large number of macromolecular protein aggregates can be generated to block the dentin pores, so the permeability is better. The biomineralization material can permeate deeply into the exposed dentin pores, with the depth of 200-500 µm (the sealing depth of the protein coating reacting with TCEP is only about 40 µm), and can reach the deep recess of the pit and fissure.

## Description

### Cross-Reference

The present application claims priority to Chinese Patent Application No. 202210109256.0, filed on January 28, 2021 and entitled "Biomineralization Material for Treating Dentin Hypersensitivity and Promoting Epitaxial Growth of Enamel, and Use thereof", which is incorporated herein by reference in its entirety.

### Technical Field

The present application relates to the technical field of biomaterials, in particular to a biomineralization material for treating dentin hypersensitivity and promoting epitaxial growth of enamel, and a use thereof.

### Background Art

According to statistics, 15.2 % to 57.4 % of adults are troubled by dentin hypersensitivity, hypersensitivity the prevalence rate of decayed teeth of children is as high as 66.9 % to 88.1 %, and the coverage rate of pit and fissure sealing services for children in 2020 is only about 22 %. Therefore, oral dental health has been receiving increasing attention.

Enamel is the hardest coat of teeth and serves as the first line of defense to protect dental health. The main ingredient of the enamel is hydroxyapatite, which is difficult to demineralize in a healthy oral environment. However, when various bacteria are wrapped by food residues for a long time, lactic acid and the like will be produced. The outermost layer of enamel is irreversibly demineralized, and then decayed teeth form and pit and fissure caries and dentin hypersensitivity are resulted. Pits and fissures are in different shapes located on deep recesses on the occlusal surfaces of teeth, of which type I, type IK and inverted type Y account for more than 50 %. Due to their special morphology, they are difficult to be cleaned thoroughly, providing stagnation sites for plaque bio-films and cariogenic matrices and ultimately leading to pit and fissure caries. Dentin hypersensitivity is mainly caused by the exposure of tooth pores on the dentin due to the exposure of the dentin resulting from an enamel defect, while external stimuli such as cold and hot, sour and peppery and pressure may penetrate through tooth pores to stimulate the pulp, causing discomfort or pain. Sealing the tooth pores is the main pathway to reduce or cure the dentin hypersensitivity, and further enamel repair can ensure that the dentin is no longer exposed, so as to achieve the effects of treating dentin hypersensitivity and decayed teeth.

Saliva contains supersaturated calcium and phosphorus ions required for remineralization, and general health products use fluoride and the like to enhance the remineralization process to treat dentin hypersensitivity and repair decayed teeth. However, this method can only be effective within a short time, and a remineralized layer has poor stability and is easy to fall off, so dentin hypersensitivity is very easy to recur, and the effect of enamel remineralization is not obvious.

In addition, biomineralization materials such as PTL/C-AMG or Lyso-PEG that have been proposed so far often require tris(2-carboxyethyl)phosphine hydrochloride (TCEP), which is a disulfide bond reducing agent with biosafety concerns, to participate in reactions. TCEP may cause adverse stimuli to user's oral tissues and is harmful to human body, which is only limited to scientific research by professionals, and shall not be used for clinical diagnosis or treatment, let alone food or medicine. In addition, lysozyme or PEGylated lysozyme reacts with TCEP to produce macromolecular protein aggregates, resulting in reduced permeability. These biomineralization coatings also require special customization of an expensive amelogenin peptide (C-AMG), which may be costly. The reported remineralization system in which amorphous calcium phosphate (ACP) is fixed by triethylamine also requires odorous and toxic triethylamine to participate, which is limited by clinical use.

At present, pit and fissure sealants are mainly polymerized resin pit and fissure sealants, but their structures are far from the enamel itself, and untight edges and high shedding rate are the main drawbacks of the existing pit and fissure sealants. Therefore, deep sealing of epitaxial growth of enamel and remineralization of hydroxyapatite (HAp) are the most ideal methods for pit and fissure sealing. How to make a biomineralization material penetrate deeply into the tooth pores or pits and fissures and generate a remineralized HAp layer are two major technical problems that need to be solved in clinical practice.

### Summary of the Invention

### Object of the present invention

In order to overcome the above defects, an object of the present application is to provide a biomineralization material for treating dentin hypersensitivity and promoting epitaxial growth of enamel, and a use thereof.

The present application proposes for the first time that a disulfide bond exchanger (e.g., cysteine (Cys)) without any toxicity may react with a film-forming protein or PEGylated film-forming protein. When a protein nano-coating with a mineralization regulating function is formed, a large number of macromolecular protein aggregates cannot be generated to block dentin pores, so the permeability is better. The biomineralization material can permeate deeply into the exposed dentin pores, with a depth of about 200 µm (the sealing depth of the protein coating reacting with TCEP is only about 40 µm), and can reach the deep recess of the pit and fissure. Through electrostatic attraction, hydrophobic interaction, van der Waals force and other effects, a protein coating capable of regulating mineralization may attract supersaturated calcium and phosphorus ions in saliva to regulate the remineralization process, form a regenerated HAp layer, seal the tooth pores, and repair enamel to treat decayed teeth and pit and fissure caries. A protein coating capable of regulating remineralization proposed in the present application does not require special treatment of proteins, nor does it require special customization of expensive amelogenin peptides. This protein coating is colorless and transparent, simple to prepare, inexpensive, and non-toxic, and has excellent biocompatibility. The remineralized HAp layer has a dense structure and strong stability. The mineralization depth can penetrate deeply into the dentin pores and deep recess of pit and fissure at 200 µm. The biomineralization material in the present application can effectively treat dentin hypersensitivity, decayed teeth and pit and fissure sealing for a long time, and is of great significance in clinical application.

### Solution

In order to fulfill the above objects of the present application, the present application adopts the following technical solutions.

In a first aspect, the present application provides a biomineralization material for treating dentin hypersensitivity and promoting epitaxial growth of enamel, wherein the biomineralization material includes the following raw materials in parts by weight: 5-72 parts of a film-forming protein or a PEGylated film-forming protein, 4-67 parts of a water-soluble disulfide bond exchanger and 1-21 parts of a pH regulator.

Further, the biomineralization material includes the following raw materials in parts by weight: 30-70 parts of a film-forming protein or a PEGylated film-forming protein, 20-67 parts of a water-soluble disulfide bond exchanger and 4-21 parts of a pH regulator; and optionally, 30-50 parts of a film-forming protein or a PEGylated film-forming protein, 10-50 parts of a water-soluble disulfide bond exchanger and 6-20 a parts of pH regulator.

Further, the water-soluble disulfide bond exchanger is a substance which has sulfydryl and is capable of undergoing a mercaptan disulfide bond exchange reaction with a protein, optionally one or more of cysteine and glutathione.

Further, the film-forming protein is one or more of lysozyme, bovine serum albumin, human serum albumin, whey albumin, insulin, α-lactalbumin, fibrinogen, β-lactoglobulin, ribonuclease A, cytochrome c, α-amylase, pepsin, myoglobin, albumin, collagen, keratin, soy protein, hemoglobin, DNA polymerase, casein, lactoferrin, trypsin, chymotrypsin, thyroglobulin, transferrin, fibrinogen, goat serum, fetal calf serum, mouse serum, immunoglobulin, milk protein, ovalbumin, concanavalin, fish skin collagen, superoxide dismutase, pancreatic lipase, laccase, histone, collagenase, cellulase, glutelin, mucin, transglutaminase, and β-galactosidase;

The PEGylated film-forming protein includes one or more of PEGylated lysozyme, PEGylated bovine serum albumin, PEGylated human serum albumin, PEGylated whey albumin, PEGylated insulin, PEGylated α-lactalbumin, PEGylated fibrinogen, PEGylated β-lactoglobulin, PEGylated ribonuclease A, PEGylated cytochrome c, PEGylated α-amylase, PEGylated pepsin, PEGylated myoglobin, PEGylated albumin, PEGylated collagen, PEGylated keratin, PEGylated soy protein, PEGylated hemoglobin, PEGylated DNA polymerase, PEGylated casein, PEGylated lactoferrin, PEGylated trypsin, PEGylated chymotrypsin, PEGylated thyroglobulin, PEGylated transferrin, PEGylated fibrinogen, PEGylated goat serum, PEGylated fetal calf serum, PEGylated mouse serum, PEGylated immunoglobulin, PEGylated milk protein, PEGylated ovalbumin, PEGylated concanavalin, PEGylated fish skin collagen, PEGylated superoxide dismutase, PEGylated pancreatic lipase, PEGylated laccase, PEGylated histone, PEGylated collagenase, PEGylated cellulase, PEGylated glutelin, PEGylated mucin, PEGylated transglutaminase, and PEGylated β-galactosidase;

Optionally, a number-average molecular weight of polyethylene glycol used in the PEGylated film-forming protein is 200-20000, and optionally, the PEGylated film-forming protein is lysozyme-PEG6000, lysozyme-PEG4000, and lysozyme-PEG2000, and preferably lysozyme-PEG2000.

Optionally, the pH adjuster is selected from one or more of sodium carbonate, sodium bicarbonate, potassium carbonate, potassium bicarbonate, dipotassium hydrogen phosphate, disodium hydrogen phosphate, sodium benzoate and sodium citrate; optionally selected from any one or more of sodium carbonate, sodium bicarbonate, potassium carbonate, potassium bicarbonate, dipotassium hydrogen phosphate, and disodium hydrogen phosphate; optionally selected from one or more of sodium carbonate, sodium bicarbonate, potassium carbonate, and potassium bicarbonate; or selected from one or both of sodium carbonate and sodium bicarbonate.

In another aspect, there is provided a mouthwash for treating dentin hypersensitivity and promoting epitaxial growth of enamel, including the biomineralization material for treating dentin hypersensitivity and promoting epitaxial growth of enamel, optionally including a buffer and an additive for diluting the biomineralization material.

Further, the biomineralization material, the HEPES buffer and the additive are uniformly mixed according to a mass-volume-mass ratio of 5-25 mg: 10-200 mL: 5-15 mg to prepare the mouthwash; Optionally, in the mouthwash, the biomineralization material has a concentration greater than 0.2 mg/mL; optionally, the biomineralization material has a concentration greater than 0.25 mg/mL; optionally, the biomineralization material has a concentration greater than 0.3 mg/mL; optionally, the biomineralization material has a concentration greater than 0.5 mg/mL; optionally, the film-forming protein or PEGylated film-forming protein in the mouthwash has a concentration not less than 0.2 mg/mL;

Optionally, the concentration of the HEPES buffer ranges from 5 mM to 20 mM, and optionally 10 mM; the additive is selected from one or more of ethanol, sodium citrate, sodium saccharin, caprylyl glycol and sorbinose; and optionally, the pH value of the mouthwash is 7-7.5.

In yet another aspect, there is provided a tooth desensitizer for promoting epitaxial growth of enamel, including a HEPES buffer and the biomineralization material for treating dentin hypersensitivity and promoting epitaxial growth of enamel;

Optionally, the biomineralization material and the HEPES buffer are prepared into a 0.1-320 mg/mL tooth desensitizer solution; optionally, the concentration is 0.2-320 mg/mL; optionally, the concentration is 0.3-320 mg/mL; optionally, the concentration is 0.5-320 mg/mL; optionally, the concentration is 1-320 mg/mL; optionally, the concentration of the film-forming protein or PEGylated film-forming protein in the tooth desensitizer is not less than 0.2 mg/mL; and Optionally, the HEPES buffer has a concentration of 5 mM-20 mM, and optionally 10 mM; optionally, the pH of the tooth desensitizer solution is 7-7.5; and optionally, a use method of the tooth desensitizer includes: dipping the tooth desensitizer with a cotton swab and uniformly coating the tooth desensitizer on the dentin, or soaking the dentin in the tooth desensitizer solution for 1-5 minutes by using a mold.

In still another aspect, there is provided a toothpaste for treating dentin hypersensitivity and promoting epitaxial growth of enamel, including a toothpaste excipient and the biomineralization material for treating dentin hypersensitivity and promoting epitaxial growth of enamel; optionally, a mass ratio of the biomineralization material to the toothpaste excipient is 1: 2-5; and optionally, the toothpaste excipient is selected from any one or more of an abrasive, a moisturizing agent, a thickener, a preservative, a pigment and an essence.

In still another aspect, there is provided a pit and fissure sealant for treating dentin hypersensitivity and promoting epitaxial growth of enamel, including the biomineralization material for treating dentin hypersensitivity and promoting epitaxial growth of enamel; the biomineralization material is uniformly mixed with a HEPES buffer according to a mass ratio of 1: 0.1-5 to prepare the pit and fissure sealant; optionally, the HEPES buffer has a concentration of 5 mM-20 mM, and optionally 10 mM; and optionally, the pH of the pit and fissure sealant solution is 7-7.5.

### Beneficial effects

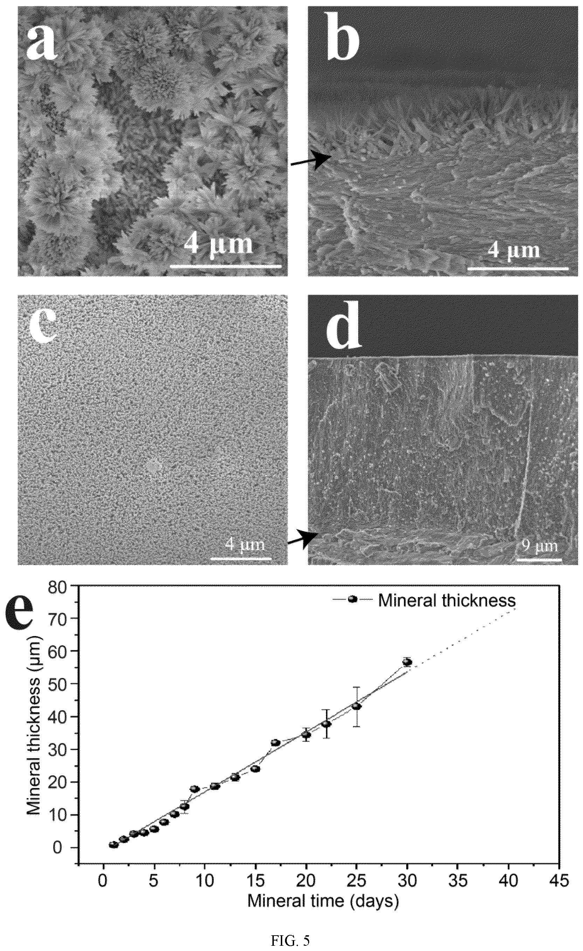

(1) According to the present application, a disulfide bond exchanger (e.g., cysteine (Cys)) without any toxicity may react with a film-forming protein or PEGylated film-forming protein, forming a protein nano-coating with a mineralization regulating function. A large number of macromolecular protein aggregates cannot be generated to block dentin pores in the mineralization regulating process of this protein nano-coating, so the permeability is better. The biomineralization material can permeate deeply into the exposed dentin pores, with a depth of about 200 µm. Through electrostatic attraction, hydrophobic interaction, van der Waals force and other effects, supersaturated calcium and phosphorus ions in saliva can be attracted to regulate the remineralization process, and form an HAp layer having certain tendencies and strong stability within a short time.
(2) The biomineralization material of the present application does not require special treatment of the protein in the process of forming the protein coating, nor does it require special customization of an expensive amelogenin peptide. The protein coating capable of regulating the remineralization proposed in the present application is colorless and transparent, simple to prepare, inexpensive, and non-toxic, and has excellent biocompatibility. The remineralized HAp layer has a dense structure and strong stability. The mineralization depth can penetrate deeply into the dentin pores and the deep recess of pit and fissure at 200 µm.
(3) The film-forming protein or PEGylated film-forming protein of the present application undergoes phase transformation with cysteine in the oral cavity to form a two-dimensional nano-sized protein film on the teeth (solid-liquid interface). This film has a large number of functional groups on the surface, which can adsorb the supersaturated calcium and phosphate ions in saliva on the surface of the film through electrostatic attraction, hydrophobic interaction, van der Waals force and other effects to form nucleation sites. In oral saliva, the insides of the dentin pores and the surface of the defected enamel are remineralized to generate a remineralized coating, so as to achieve the purposes of deep sealing of the dentin pores to treat dentin hypersensitivity, and repair enamel to prevent decayed teeth and pit and fissure sealing. With the increase of mineralization time, the sealing depth of the dentin pores may reach about 500 µm, and the thickness of enamel remineralization may also increase linearly with the mineralization time, as shown in FIG. 5. Surface-remineralized hydroxyapatite has a certain tendency and has properties comparable to those of natural enamel.
(4) The biomineralization material of the present application may be used to prepare a tooth desensitizer, a mouthwash, a multifunctional toothpaste and a pit and fissure sealant. According to the tooth desensitizer, the mouthwash, the multifunctional toothpaste and the pit and fissure sealant in the present application, film-forming protein molecules or PEGylated film-forming protein molecules are used to undergo a phase transition reaction with a water-soluble disulfide bond exchanger in the dentin pores, on the surface of the defected enamel and inside the pit and fissure through surface interface induction for self-assembly to form a protein nano-film capable of regulating remineralization with a thickness of about 20-45 nm. This film is colorless and transparent, has good adhesion and also has a function of strengthening the remineralization of the enamel surface to produce new hydroxyapatite.
(5) The main ingredient used in the present application is protein, which can effectively seal the dentin pores to treat dentin hypersensitivity, effectively repair enamel, prevent acid erosion and demineralization of enamel, and prevent the formation of decayed teeth and pit and fissure caries. The present application focuses on the sealing depth of the tooth pores of the dentin, as well as the remineralization effect and the sealing effect of the enamel surface and the inside of the pit and fissure. Because the dentin hypersensitivity is caused by the stimulation to dental nerves by the exposure of the pore from the dentin, decayed teeth and pit and fissure caries are initially manifested as demineralization of the enamel surface. Therefore, the deep sealing of the inside of the tooth pores of the dentin and the epitaxial growth and remineralization of the enamel surface can prevent and repair the caries before a permanent structural damage of the tooth or after the occurrence of the permanent structural damage, the deep sealing of the tooth pores of the dentin can be realized to treat dentin hypersensitivity and remineralization of enamel, repair the enamel defect, and further prevent the further formation of decayed teeth or pit and fissure caries. After a period of use of this tooth desensitizer, mouthwash, multi-purpose toothpaste or pit and fissure sealant, the surface of the demineralized enamel and the inside of the dentin pores can be remineralized, or normal teeth are mineralized, thereby preventing decayed teeth and inhibiting dentin hypersensitivity. The contact time between the tooth desensitizer, mouthwash, multi-functional toothpaste or pit and fissure sealant and teeth should be about 20 s to 2 min. During the use time, a remineralization-regulated two-dimensional protein nano-film may be formed inside the dentin pores, on the enamel surface and deep recess of pit and fissure, and then the inside of the dentin pores, the enamel surface and the deep recess of the pit and fissure may be remineralized in oral saliva to form an HAp layer.
(6) The tooth desensitizer, mouthwash, multifunctional toothpaste or pit and fissure sealant prepared by the biomineralization material of the present application take a protein as a main component, and the water-soluble disulfide bond exchanger is also a protein and non-toxic, which meets the FDA's definition of safe substances, is used for Class II medical devices, without any harm to the human body, and has excellent biocompatibility. The biomineralization material of the present application may also be made into other products for tooth desensitization and prevention of enamel demineralization.

### Brief Description of the Drawings

One or more examples are illustrated by pictures in the corresponding drawings, and these illustrative descriptions do not constitute a limitation to the examples. The word "exemplary" used herein means "to be used as an instance, an example, or being illustrative". Any example illustrated herein as "exemplary" is unnecessarily construed as superior to or better than other examples.

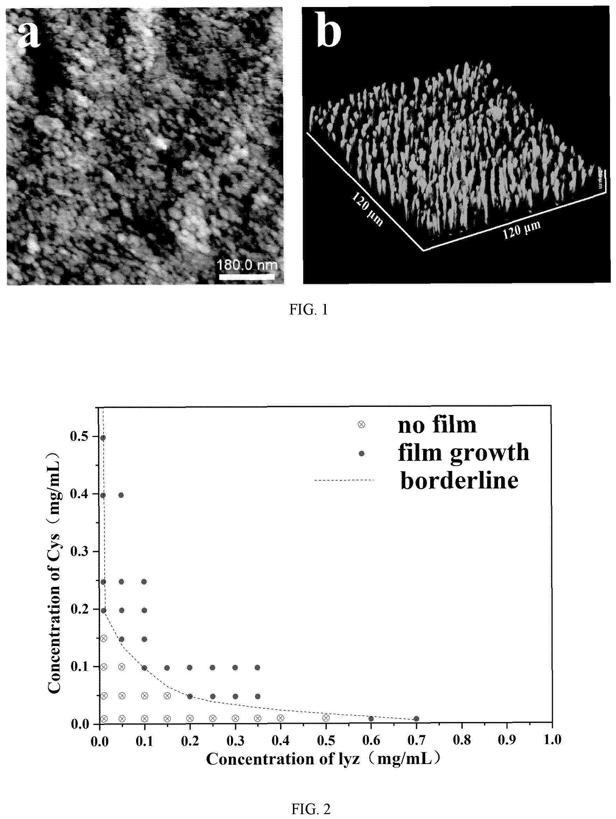

FIG. 1 shows nano-film and deep film-forming diagrams formed by a mouthwash in Test Example 1 and a tooth desensitizer in Test Example 2 respectively on the enamel surface and inside the dentin pores in the present application. Wherein, a is an atomic force map of a two-dimensional nano-film of a biological protein formed on the enamel surface after gargling with a mouthwash corresponding to Example 1; and b is a laser confocal three-dimensional imaging image of a tooth desensitizer corresponding to Example 1 inducing deep film formation inside dentin pores.
FIG. 2 shows a film-forming critical line of Lyzyme (Lyz) concentration v.s. cysteine (Cys) concentration under the influence of binary factors in Test Example 3 of the present application.

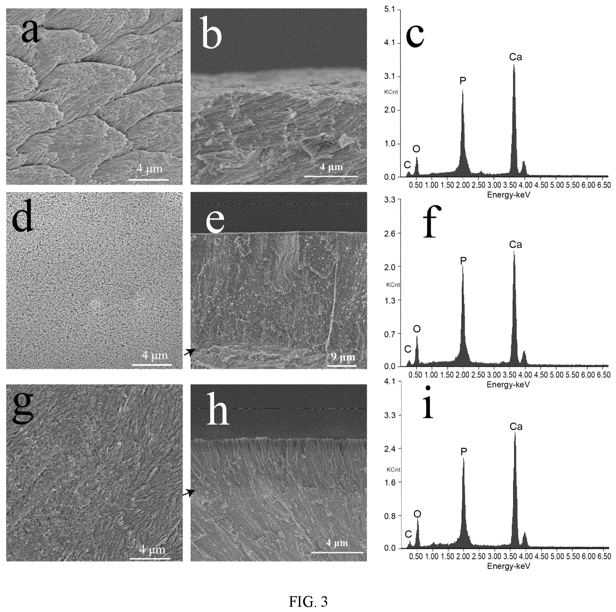

FIG. 3 shows scanning electron microscope pictures of a new remineralized coating on the enamel surface tested by in vitro test of the mouthwash in Test Example 1 of the present application. Wherein, a is an acid-etched enamel surface; b is an enamel cross-section; c is element energy spectrum analysis of blank enamel; d is an enamel surface map two weeks after using the mouthwash corresponding to Example 1 and inducing mineralization in the modified artificial saliva; e is an enamel cross-sectional view two weeks after using the mouthwash corresponding to Example 1 and inducing mineralization in modified artificial saliva; f is an energy spectrum analysis diagram of the enamel surface two weeks after using the mouthwash corresponding to Example 1 and inducing mineralization in the modified artificial saliva; g is an enamel surface diagram three days after using the mouthwash corresponding to Example 1 and inducing mineralization in real oral saliva; h is an enamel cross-sectional view three days after using the mouthwash corresponding to Example 1 and inducing mineralization in real oral saliva; and i is an energy spectrum analysis diagram of the enamel surface three days after using the mouthwash corresponding to Example 1 and inducing mineralization in real oral saliva.

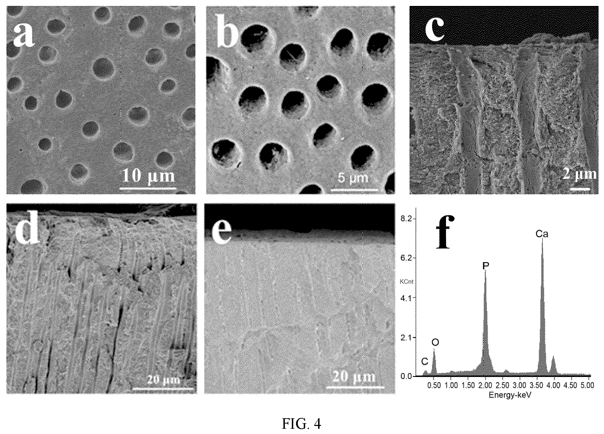

FIG. 4 shows scanning electron microscope pictures of a blank dentin slice and a dentin slice coating with a tooth desensitizer corresponding to Example 1. Wherein a is a blank dentin surface; b is a dentin surface coating with the tooth desensitizer corresponding to Example 1; c is a blank dentin cross-section; d is a dentin cross-section seven days after the biomineralization when the tooth desensitizer was not coated; e is a dentin cross-section (the deepest part reaches about 200 µm) seven days after the biomineralization when the tooth desensitizer corresponding to Example 1 was coated; and f is element energy spectrum analysis of remineralized crystals after the tooth desensitizer corresponding to Example 1 was coated.
FIG. 5 shows enamel scanning electron microscope images and change of mineralization thickness with mineralization time using a mouthwash corresponding to Comparative Example 1 and a mouthwash in Example 1. Wherein a is an enamel surface map two weeks after using the mouthwash corresponding to Comparative Example 1; b is a cross-sectional view of enamel remineralization two weeks after using the mouthwash of Comparative Example 1; c is an enamel surface diagram two weeks after using the mouthwash corresponding to Example 1 of the present application; d is a cross-sectional view of enamel remineralization two weeks after using the mouthwash corresponding to Example 1 of the present application; and e is a mineral thickness change of the mouthwash corresponding to Example 1 of the present application with the mineralization time.

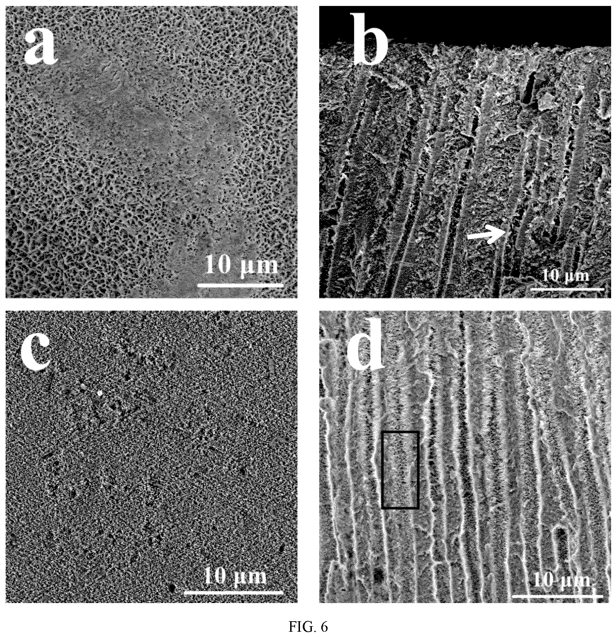

FIG. 6 shows dentin scanning electron microscope pictures using a tooth desensitizer corresponding to Comparative Example 2 and a tooth desensitizer corresponding to Example 1. Wherein a is a dentin surface diagram three weeks after using the desensitizer obtained by Comparative Example 2; b is a cross-sectional view of dentin remineralization three weeks after using the desensitizer obtained by Comparative Example 2; c is a dentin surface diagram three weeks after using the tooth desensitizer corresponding to Example 1 of the present application; and d is a cross-sectional view of dentin remineralization three weeks after using the tooth desensitizer corresponding to Example 1 of the present application.

### Detailed Description of the Invention

In order to make the objectives, technical solutions and advantages of the examples of the present application more clearly, the technical solutions in the examples of the present application will be described clearly and completely. Apparently, the described examples are merely a part of examples, rather than all examples, of the present application. Based on the examples of the present application, all other examples derived by a person of ordinary skill in the art without creative efforts shall fall within the protection scope of the present application.

In addition, in order to better illustrate the present application, numerous specific details are given in the specific embodiments below. Those skilled in the art should understand that the present application may also be implemented without certain specific details. In some examples, the raw materials, components, methods, means, etc., which are well known to those skilled in the art, are not described in detail, so as to highlight the main purpose of the present application.

Unless otherwise expressly stated, throughout the description and claims, the terms "include(s)/comprise(s)" or its transformations such as "including" or "comprising" is to be construed to include the stated element or component without excluding other elements or other components.

### Example 1

70 mg of lysozyme, 65 mg of cysteine (Cys) and 20 mg of sodium bicarbonate were mixed uniformly to obtain a biomineralization material for treating dentin hypersensitivity and promoting epitaxial growth of enamel.

### Example 2

30 mg of lysozyme, 46 mg of Cys and 15.2 mg of sodium bicarbonate were mixed uniformly to obtain a biomineralization material for treating dentin hypersensitivity and promoting epitaxial growth of enamel.

### Example 3

40 mg of lysozyme, 20.8 mg of glutathione (GSH) and 6.2 mg of sodium bicarbonate were mixed uniformly to obtain a biomineralization material for treating dentin hypersensitivity and promoting epitaxial growth of enamel.

### Example 4

52 mg of lysozyme, 65 mg of GSH and 18.2 mg of sodium bicarbonate were mixed uniformly to obtain a biomineralization material for treating dentin hypersensitivity and promoting epitaxial growth of enamel.

### Example 5

72 mg of PEGylated lysozyme, 67 mg of Cys and 20.6 mg of sodium bicarbonate were mixed uniformly to obtain a biomineralization material for treating dentin hypersensitivity and promoting epitaxial growth of enamel.

### Example 6

62 mg of PEGylated lysozyme, 50 mg of GSH and 16.2 mg of sodium bicarbonate were mixed uniformly to obtain a biomineralization material for treating dentin hypersensitivity and promoting epitaxial growth of enamel.

### Example 7

The mouthwashes in the present example were composed of the biomineralization materials respectively obtained in the above Examples 1 to 6 and a 10 mM HEPES buffer. During use, 6.2 mg of a biomineralization material and 40 mL of a 10 mM HEPES buffer were added with 5 mg of sodium saccharin and 2 mg of sorbinose and mixed uniformly to obtain a mouthwash for treating dentin hypersensitivity and promoting epitaxial growth of enamel.

### Example 8

The mouthwashes in the present example were composed of the biomineralization materials respectively obtained in the above Examples 1 to 6 and a 10 mM HEPES buffer. During use, 20 mg of a biomineralization material and 240 mL of a 10 mM HEPES buffer were added with 6 mg of sodium saccharin and 4 mg of sorbinose and mixed uniformly to obtain a mouthwash for treating dentin hypersensitivity and promoting epitaxial growth of enamel.

### Example 9

The tooth desensitizers in the present example were composed of the biomineralization materials respectively obtained in the above Examples 1 to 6 and a 10 mM HEPES buffer. During use, 35 mg of a biomineralization material and 120 mL of a 10 mM HEPES buffer were mixed uniformly to obtain a tooth desensitizer for treating dentin hypersensitivity and promoting epitaxial growth of enamel.

### Example 10

The tooth desensitizers in the present example were composed of the biomineralization materials respectively obtained in the above Examples 1 to 6 and a 10 mM HEPES buffer. During use, 52 mg of a biomineralization material and 152 mL of a 10 mM HEPES buffer were mixed uniformly to obtain a tooth desensitizer for treating dentin hypersensitivity and promoting epitaxial growth of enamel.

### Example 11

The pit and fissure sealants in the present example were composed of the biomineralization materials respectively obtained in the above Examples 1 to 6 and a 10 mM HEPES buffer. During use, 42 mg of a biomineralization material and 25 mL of a 10 mM HEPES buffer were mixed uniformly to obtain a pit and fissure sealant for treating dentin hypersensitivity and promoting epitaxial growth of enamel.

### Example 12

The pit and fissure sealants in the present example were composed of the biomineralization materials respectively obtained in the above Examples 1 to 6 and 10 mM HEPES buffer. During use, 26.2 mg of a biomineralization material and 30.2 mL of a 10 mM HEPES buffer were mixed uniformly to obtain a pit and fissure sealant for treating dentin hypersensitivity and promoting epitaxial growth of enamel.

### Example 13

The multifunctional toothpastes in the present example were composed of the biomineralization materials respectively obtained in the above Examples 1 to 6 and a toothpaste excipient. During use, 12 mg of a biomineralization material and 20 mg of a toothpaste excipient were mixed uniformly to obtain a multifunctional toothpaste for treating dentin hypersensitivity and promoting epitaxial growth of enamel.

### Example 14

The multifunctional toothpastes in the present example were composed of the biomineralization materials respectively obtained in the above Examples 1 to 6 and a toothpaste excipient. During use, 45 mg of a biomineralization material and 100 mg of a toothpaste excipient were mixed uniformly to obtain a multifunctional toothpaste for treating dentin hypersensitivity and promoting epitaxial growth of enamel.

The lysozyme or PEGylated lysozyme in the above examples may also be replaced with any one or more of bovine serum albumin, human serum albumin, whey albumin, insulin, α-lactalbumin, fibrinogen, β-lactoglobulin, ribonuclease A, cytochrome c, α-amylase, pepsin, myoglobin, albumin, collagen, keratin, soy protein, hemoglobin, DNA polymerase, casein, lactoferrin, trypsin, chymotrypsin, thyroglobulin, transferrin, fibrinogen, goat serum, fetal calf serum, mouse serum, immunoglobulin, milk protein, ovalbumin, concanavalin, fish skin collagen, superoxide dismutase, pancreatic lipase, laccase, histone, collagenase, cellulase, glutelin, mucin, transglutaminase, and β-galactosidase; or the PEGylated film-forming protein includes one or more of PEGylated lysozyme, PEGylated bovine serum albumin, PEGylated human serum albumin, PEGylated whey albumin, PEGylated insulin, PEGylated α-lactalbumin, PEGylated fibrinogen, PEGylated β-lactoglobulin, PEGylated ribonuclease A, PEGylated cytochrome c, PEGylated α-amylase, PEGylated pepsin, PEGylated myoglobin, PEGylated albumin, PEGylated collagen, PEGylated keratin, PEGylated soy protein, PEGylated hemoglobin, PEGylated DNA polymerase, PEGylated casein, PEGylated lactoferrin, PEGylated trypsin, PEGylated chymotrypsin, PEGylated thyroglobulin, PEGylated transferrin, PEGylated fibrinogen, PEGylated goat serum, PEGylated fetal calf serum, PEGylated mouse serum, PEGylated immunoglobulin, PEGylated milk protein, PEGylated ovalbumin, PEGylated concanavalin, PEGylated fish skin collagen, PEGylated superoxide dismutase, PEGylated pancreatic lipase, PEGylated laccase, PEGylated histone, PEGylated collagenase, PEGylated cellulase, PEGylated glutelin, PEGylated mucin, PEGylated transglutaminase, and PEGylated β-galactosidase.

### Comparative Example 1

20 mg of lysozyme, 10 mg of amelogenin peptide, 10 mg of disodium hydrogen phosphate, 5.2 mg of TCEP and 3.5 mg of sodium hydrogen phosphate were mixed uniformly to obtain the biomineralization material in the present comparative example.

2 mg of the biomineralization material in the present comparative example was added to 10 mL of a HEPES buffer, then added with 8 mg of sodium saccharin and 2 mg of sorbinose and mixed uniformly to obtain a mouthwash for preventing enamel demineralization in the present comparative example. The concentration of the HEPES buffer in the present comparative example was 20 mM.

### Comparative Example 2

40 mg of PEGylated lysozyme, 10 mg of calcium chloride, 10 mg of TCEP and 60 mg of sodium bicarbonate were mixed uniformly to obtain a desensitizer in the present comparative example.

60 mg of the biomineralization material in the present comparative example was added to 10 mL of deionized water, and mixed uniformly to obtain a 6 mg/mL desensitizer solution in the present comparative example.

In order to prove the beneficial effects of the present application, the inventors have carried out a performance test on the mouthwashes obtained in Example 8 for treating dentin hypersensitivity and promoting the epitaxial growth of enamel, and the specific tests were as follows.

### Test Example 1

Caries-free human third molar samples (the tooth samples were provided by the Stomatological Hospital of the Air Force Military Medical University and the Stomatological Hospital of Tianjin Medical University, and the research was approved by the Medical Ethics Committee of the above-mentioned units) were collected. After cleaning, an enamel slice having an enamel of 6 mm*6 mm*1.5 mm was treated with a SYJ160 slow microtome and 200-6000 mesh sandpaper. The polished enamel slice was etched with 37% (wt%) phosphoric acid for 30 seconds, rinsed with ultrapure water, sonicated for 15 min, blow-dried with nitrogen, then put into the mouthwashes obtained in Example 8, and soaked for 1 min. A gargling process was imitated.

A two-dimensional nano-film of a biological protein was formed on the enamel surface after 1 min, wherein the test result of a mouthwash in Example 8 (the mouthwash prepared from the biomineralization material of Example 1 according to the method of Example 8, hereinafter referred to as the mouthwash corresponding to Example 1) was shown in FIG. 1a, and its thickness was about 30-50 nm. Mineralization conditions in modified artificial saliva and real oral saliva were verified respectively.
1) The above-mentioned enamel covered with the protein film was soaked in modified artificial saliva and biomineralized at 37 °C, and the modified artificial saliva was replaced once a day. In the mineralization process, the enamel was removed from the modified artificial saliva every day, rinsed with ultrapure water, blow-dried with nitrogen, soaked in the mouthwash obtained in Example 8 for 1 min, and then transferred to the modified artificial saliva. This process was repeated twice a day. Two weeks later, the plane morphology of a remineralized coating of enamel was observed with a scanning electron microscope, and surface scanning of the remineralized coating was carried out with an energy spectrometer. The elements of the remineralized coating were analyzed. The enamel was crushed by the energy spectrometer. The cross-sectional morphology of the remineralized coating of the enamel was observed with a scanning electron microscope, wherein the scanning results of the energy spectrometer corresponding to the mouthwash corresponding to Example 1 were shown in FIGs. 3d and 3f, and the scanning results of the energy spectrometer were shown in FIG. 3e.
   According to the results shown in FIG. 3, the two-dimensional nano-film of the biological protein grew on the enamel by an in-situ growth method. The enamel coated with the protein film may simulate biomineralization in the modified artificial saliva to generate well-oriented hydroxyapatite having a calcium-phosphorus ratio consistent with a calcium-phosphorus ratio of natural enamel. New hydroxyapatite was remineralized on the acid-etched enamel surface (FIGs. 3a-c) to prevent enamel demineralization and decayed teeth.
2) The above-mentioned enamel on which a protein film grew was soaked in real oral saliva after centrifugation (wherein the real saliva was taken from healthy adult volunteers, samples were taken at least 2 h after meals, and the sampling was approved by the Medical Ethics Committee), and the enamel on which the film grew was soaked in real oral saliva to simulate biomineralization. After three days, the surface morphology of the enamel was observed, and the results were shown in FIGs. 3g-i. As can be seen from the drawings, the enamel on which the protein film grew was soaked in real oral saliva, and regenerated hydroxyapatite with a high degree of orientation could be remineralized, which proved that the protein film in the oral saliva environment may also play a role in mineralization and could be applied to the oral environment of the human body.

The inventors further compared film-formed and mineralized enamel scanning electron microscope images of the mouthwash of Comparative Example 1 and the mouthwash corresponding to Example 1 according to the above method, and the results were shown in FIG. 5. The results showed that when the mouthwash corresponding to Example 1 was used, the surface of the formed mineralized layer was dense (FIG. 5c). The thickness of the remineralized layer of the enamel may increase linearly with the mineralization time (FIG. 5e), while the surface of the mineralized layer of the enamel of the mouthwash of Comparative Example 1 was not dense (FIG. 5a); and the effect on the thickness of the remineralized layer of the enamel of the mouthwash of Comparative Example 1 (FIG. 5b, a position where an arrow points) was also far less than the mineral depth of the mouthwash corresponding to Example 1 (FIG. 5d, a position where an arrow points).

### Test Example 2

In order to prove the beneficial effects of the present application, the inventors have carried out a performance test on the tooth desensitizers obtained in Example 10, and the specific tests were as follows.

Caries-free human third molar samples (the tooth samples were provided by the Stomatological Hospital of the Air Force Military Medical University and the Stomatological Hospital of Tianjin Medical University, and the research was approved by the Medical Ethics Committee of the above-mentioned units) were collected. After cleaning, a dentin slice having a thickness of 2 mm was prepared with a SYJ160 slow microtome and polished to obtain a dentin sample of 5 mm×5 mm×2 mm. The dentin sample was etched with 17% (wt%) EDTA solution and 2% (wt%) NaClO solution as a test sample.

The above sample was soaked in a tooth desensitizer solution and placed at 37 °C for 2 min before being removed. The tooth desensitizer may be specifically bound with fluorescent dye thiosulfurin T (ThT), and a three-dimensional imaging test could be used by a laser confocal microscope to prove that this tooth desensitizer could penetrate into the dentin pores to form a film, wherein the test result of the tooth desensitizer in Example 10 (the tooth desensitizer prepared from the biomineralization material of Example 1 in accordance with the method of Example 10, hereinafter referred to as the tooth desensitizer corresponding to Example 1) was shown in FIG. 1b.

The dentin slice coated with the tooth desensitizer was placed in a 12-well plate, added with the modified artificial saliva and cultured at 37 °C. The modified artificial saliva was replaced every day. After remineralization for seven days, the sealing status of the dentin pores was observed by scanning electron microscopy. A blank dentin slice that was not coated with the tooth desensitizer was used as a control experiment, and the test results of the tooth desensitizer corresponding to Example 1 were shown in FIG. 4.

As can be seen from FIG. 4e, the inside of the tooth pores of the dentin slice on which the tooth desensitizer was used to form a protein film was sealed by remineralized crystals, while the pores of the dentin slice in the blank control group which was not coated with the tooth desensitizer (FIG. 4d) had no crystals inside, and the dentin remained exposed. Element energy spectrum analysis (FIG. 4f) proved that the remineralized crystals were hydroxyapatite HAp. This test example showed that the tooth desensitizer proposed in the present application could penetrate deeply into the dentin pores to form a protein nano-coating with a remineralization regulating function, could induce the remineralization of dentin in the modified artificial saliva environment, and deeply seal the tooth pores of the dentin, so as to treat the symptoms of dentin hypersensitivity.

The inventors further extended the remineralization time (three weeks) according to the above method and compared the dentin scanning electron microscope pictures of mineralization of the tooth desensitizer of Comparative Example 2 and the tooth desensitizer corresponding to Example 1, and the results were shown in FIG. 6. The results showed that with the increase of the mineralization time, the sealing depth of the dentin pores of the tooth desensitizer corresponding to Example 1 could reach 200-500 µm (the square in FIG. 6d was about 420 µm from the surface of the dentin slice), while the sealing depth of the dentin pores of the tooth desensitizer of Comparative Example 2 was only about 40 µm (at a white arrow in FIG. 6b).

### Test Example 3

In order to study a film-forming critical line between a film-forming protein and cysteine, the inventors prepared different concentrations of film-forming protein solutions and cysteine solutions, and curves were drawn as shown in FIG. 2. FIG. 2 showed that under the concentrations of Lyz and Cys below a boundary line (left), these solutions could not be aggregated to form a complete two-dimensional nano-film with a remineralization regulating process. As can be seen from FIG. 2, when the protein concentration was greater than or equal to 0.2 mg/mL, a small amount of cysteine could form a film. However, when the protein concentration was 0.2 mg/mL, higher concentration of cysteine was required for film formation.

In summary, the biomineralization material used in the present application comprises mainly a protein, etc. The material has no toxicity and excellent biocompatibility, simple preparation, and convenient use. In vitro tests had proved that the remineralized crystal structure was stable and comparable to natural enamel or dentin, which could desensitize teeth for a long time and had excellent treatment effects. The biomineralization material used in the present application for treating dentin hypersensitivity and repairing enamel defects to prevent decayed teeth, as well as the products such as the tooth desensitizer, mouthwash and toothpaste prepared from the mineralized material have excellent therapeutic prospects.

According to the present application, a protein nano-film is formed from a protein lysozyme (or PEGylated lysozyme) under the action of a disulfide bond exchanger (cysteine or glutathione). This nano-film could be used for the remineralization of dentin and the promotion of epitaxial growth of enamel for the treatment of dentin hypersensitivity, caries and pit and fissure sealing. The biomineralization material in the present application could penetrate deeply into the exposed dentin pores, the enamel surface of caries and pits and fissures by simple soaking, smearing and other ways to form a protein nano-film coating with a function of inducing remineralization, which could induce saturated calcium and phosphorus ions in saliva to remineralize inside the dentin pores to form an HAp layer, and promote the remineralization of HAp on the enamel surface so that the enamel grew epitaxially, so as to achieve the purposes of treating dentin hypersensitivity, and preventing caries and pit and fissure sealing. The protein coating formed in the present application inside the dentin and on the enamel surface also has antibacterial and anti-fouling abilities, which are essential for ensuring oral cleanliness and health. The biomineralization materials mentioned in the present application are all safe substances recognized by the United States Food and Drug Administration (FDA), has better biocompatibility, and has broader prospects for clinical applications.

It should be eventually noted that: the above examples are merely used to illustrate the technical solutions of the present application, but are not limited thereto. Although the present application is described in detail with reference to the above examples, a person of ordinary skill in the art should understand: the technical solutions described in the foregoing examples may be modified, or some of the technical features may be equivalently replaced. However, these modifications and substitutions do not make the corresponding technical solutions essentially depart from the scope of the technical solutions in the examples of the present application.

### Industrial Applicability

The present application provides a biomineralization material for treating dentin hypersensitivity and promoting epitaxial growth of enamel and a use thereof. The biomineralization material includes the following raw materials in parts by weight: 5-72 parts of a film-forming protein or a PEGylated film-forming protein, 4-67 parts of a water-soluble disulfide bond exchanger and 1-21 parts of a pH regulator. The tooth desensitizer, mouthwash, multifunctional toothpaste or pit and fissure sealant prepared by the biomineralization material of the present application take a protein as a main component, and the water-soluble disulfide bond exchanger is also a protein and non-toxic, which has no any harm to the human body, and has excellent biocompatibility. The biomineralization material of the present application may also be made into other products for tooth desensitization and prevention of enamel demineralization.

## Claims

1. A biomineralization material for treating dentin hypersensitivity and promoting epitaxial growth of enamel, comprising the following raw materials in parts by weight: 5-72 parts of a film-forming protein or a PEGylated film-forming protein, 4-67 parts of a water-soluble disulfide bond exchanger and 1-21 parts of a pH regulator.

2. The biomineralization material for treating dentin hypersensitivity and promoting epitaxial growth of enamel according to claim 1, comprising the following raw materials in parts by weight: 30-72 parts of a film-forming protein or a PEGylated film-forming protein, 20-67 parts of a water-soluble disulfide bond exchanger and 4-21 parts of a pH regulator; and optionally, 30-50 parts of a film-forming protein or a PEGylated film-forming protein, 10-50 parts of a water-soluble disulfide bond exchanger and 6-20 parts of a pH regulator.

3. The biomineralization material for treating dentin hypersensitivity and promoting epitaxial growth of enamel according to claim 1, wherein the water-soluble disulfide bond exchanger is a substance which has sulfydryl and is capable of undergoing a mercaptan disulfide bond exchange reaction with a protein, optionally one or more of cysteine and glutathione.

4. The biomineralization material for treating dentin hypersensitivity and promoting epitaxial growth of enamel according to any one of claims 1-3, wherein the film-forming protein is any one or more of lysozyme, bovine serum albumin, human serum albumin, whey albumin, insulin, α-lactalbumin, fibrinogen, β-lactoglobulin, ribonuclease A, cytochrome c, α-amylase, pepsin, myoglobin, albumin, collagen, keratin, soy protein, hemoglobin, DNA polymerase, casein, lactoferrin, trypsin, chymotrypsin, thyroglobulin, transferrin, fibrinogen, goat serum, fetal calf serum, mouse serum, immunoglobulin, milk protein, ovalbumin, concanavalin, fish skin collagen, superoxide dismutase, pancreatic lipase, laccase, histone, collagenase, cellulase, glutelin, mucin, transglutaminase, and β-galactosidase;
the PEGylated film-forming protein comprises any one or more of PEGylated lysozyme, PEGylated bovine serum albumin, PEGylated human serum albumin, PEGylated whey albumin, PEGylated insulin, PEGylated α-lactalbumin, PEGylated fibrinogen, PEGylated β-lactoglobulin, PEGylated ribonuclease A, PEGylated cytochrome c, PEGylated α-amylase, PEGylated pepsin, PEGylated myoglobin, PEGylated albumin, PEGylated collagen, PEGylated keratin, PEGylated soy protein, PEGylated hemoglobin, PEGylated DNA polymerase, PEGylated casein, PEGylated lactoferrin, PEGylated trypsin, PEGylated chymotrypsin, PEGylated thyroglobulin, PEGylated transferrin, PEGylated fibrinogen, PEGylated goat serum, PEGylated fetal calf serum, PEGylated mouse serum, PEGylated immunoglobulin, PEGylated milk protein, PEGylated ovalbumin, PEGylated concanavalin, PEGylated fish skin collagen, PEGylated superoxide dismutase, PEGylated pancreatic lipase, PEGylated laccase, PEGylated histone, PEGylated collagenase, PEGylated cellulase, PEGylated glutelin, PEGylated mucin, PEGylated transglutaminase, and PEGylated β-galactosidase; and
optionally, a number-average molecular weight of polyethylene glycol used in the PEGylated film-forming protein is 200-20000, and optionally, the PEGylated film-forming protein is lysozyme-PEG6000, lysozyme-PEG4000, and lysozyme-PEG2000, and preferably lysozyme-PEG2000.

5. The biomineralization material for treating dentin hypersensitivity and promoting epitaxial growth of enamel according to claim 1, wherein the pH adjuster is selected from one or more of sodium carbonate, sodium bicarbonate, potassium carbonate, potassium bicarbonate, dipotassium hydrogen phosphate, disodium hydrogen phosphate, sodium benzoate and sodium citrate; optionally selected from any one or more of sodium carbonate, sodium bicarbonate, potassium carbonate, potassium bicarbonate, dipotassium hydrogen phosphate, and disodium hydrogen phosphate; optionally selected from one or more of sodium carbonate, sodium bicarbonate, potassium carbonate, and potassium bicarbonate; or optionally selected from one or both of sodium carbonate and sodium bicarbonate.

6. A mouthwash for treating dentin hypersensitivity and promoting epitaxial growth of enamel, comprising the biomineralization material for treating dentin hypersensitivity and promoting epitaxial growth of enamel according to any one of claims 1-5, and optionally comprising a buffer and an additive for diluting the biomineralization material.

7. The mouthwash for treating dentin hypersensitivity and promoting epitaxial growth of enamel according to claim 6, wherein the biomineralization material, the HEPES buffer and the additive are uniformly mixed according to a mass-volume-mass ratio of 5-25 mg: 10-200 mL: 5-15 mg to prepare the mouthwash;
optionally, in the mouthwash, the biomineralization material has a concentration greater than 0.2 mg/mL; optionally, the biomineralization material has a concentration greater than 0.25 mg/mL; optionally, the biomineralization material has a concentration greater than 0.3 mg/mL; optionally, the biomineralization material has a concentration greater than 0.5 mg/mL; optionally, the film-forming protein or PEGylated film-forming protein in the mouthwash has a concentration not less than 0.2 mg/mL; and
optionally, the concentration of the HEPES buffer ranges from 5 mM to 20 mM, and optionally 10 mM; the additive is selected from one or more of ethanol, sodium citrate, sodium saccharin, caprylyl glycol and sorbinose; and optionally, the pH value of the mouthwash is 7-7.5.

8. A tooth desensitizer for promoting epitaxial growth of enamel, comprising a HEPES buffer and the biomineralization material for treating dentin hypersensitivity and promoting epitaxial growth of enamel according to any one of claims 1 to 5;
optionally, the biomineralization material and the HEPES buffer are prepared into a 0.1-320 mg/mL tooth desensitizer solution; optionally, the concentration is 0.2-320 mg/mL; optionally, the concentration is 0.3-320 mg/mL; optionally, the concentration is 0.5-320 mg/mL; optionally, the concentration is 1-320 mg/mL; optionally, the concentration of the film-forming protein or PEGylated film-forming protein in the tooth desensitizer is not less than 0.2 mg/mL; and
optionally, the HEPES buffer has a concentration of 5 mM-20 mM, and optionally 10 mM; optionally, the pH of the tooth desensitizer solution is 7-7.5; and optionally, a use method of the tooth desensitizer comprises: dipping the tooth desensitizer with a cotton swab and uniformly coating the tooth desensitizer onto the dentin, or soaking the dentin in the tooth desensitizer solution for 1-5 minutes by using a mold.

9. A toothpaste for treating dentin hypersensitivity and promoting epitaxial growth of enamel, comprising a toothpaste excipient and the biomineralization material for treating dentin hypersensitivity and promoting epitaxial growth of enamel according to any one of claims 1-5; optionally, a mass ratio of the biomineralization material to the toothpaste excipient being 1: 2-5; and optionally, the toothpaste excipient is selected from any one or more of an abrasive, a moisturizing agent, a thickener, a preservative, a pigment and an essence.

10. A pit and fissure sealant for treating dentin hypersensitivity and promoting epitaxial growth of enamel, comprising the biomineralization material for treating dentin hypersensitivity and promoting epitaxial growth of enamel according to any one of claims 1-5; the biomineralization material is uniformly mixed with a HEPES buffer according to a mass ratio of 1: 0.1-5 to prepare the pit and fissure sealant; optionally, the HEPES buffer has a concentration of 5 mM-20 mM, and optionally 10 mM; and optionally, the pH of the pit and fissure sealant solution is 7-7.5.
